(19) Europäisches Patentamt / European Patent Office / Office européen des brevets

(11) **EP 2 094 637 B1**

(12) **EUROPEAN PATENT SPECIFICATION**

(45) Date of publication and mention
of the grant of the patent:
**31.10.2012 Bulletin 2012/44**

(21) Application number: **07856926.6**

(22) Date of filing: **19.12.2007**

(51) Int Cl.:
*C07C 51/41* (2006.01)     *C07C 55/02* (2006.01)
*C07C 55/16* (2006.01)     *C07C 55/18* (2006.01)
*C08K 5/098* (2006.01)     *C08L 23/12* (2006.01)

(86) International application number:
**PCT/EP2007/011203**

(87) International publication number:
**WO 2008/074494 (26.06.2008 Gazette 2008/26)**

(54) **BETA-NUCLEATING AGENT FOR POLYPROPLYENE AND PROCESS FOR ITS PREPARATION**

BETA-NUKLEIERUNGSMITTEL FÜR POLYPROPYLEN UND VERFAHREN ZU DESSEN HERSTELLUNG

AGENT DE BETA-NUCLÉATION POUR LE POLYPROPYLÈNE ET SON PROCÉDÉ DE PRÉPARATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.12.2006 EP 06026328**

(43) Date of publication of application:
**02.09.2009 Bulletin 2009/36**

(73) Proprietor: **Borealis Technology OY**
**06101 Porvoo (FI)**

(72) Inventors:
• **WOLFSCHWENGER, Johannes**
**4491 Niederneukirchen (AT)**
• **GAHLEITNER, Markus**
**4501 Neuhofen/Krems (AT)**

(74) Representative: **Grünecker, Kinkeldey,**
**Stockmair & Schwanhäusser**
**Leopoldstrasse 4**
**80802 München (DE)**

(56) References cited:
**EP-A1- 0 682 066     WO-A-02/078924**
**US-A- 5 231 126**

**Description**

The present invention concerns a novel method for preparing a β-nucleating agent for polypropylene, a novel β-nucleating agent obtained thereby and a polypropylene composition comprising this β-nucleating agent.

Prior art

Polypropylene crystallizes typically when cooling down a melt in the monoclinic α-modification. In addition to this α-modification, polypropylene, however, may also crystallize in the hexagonal β-modification and the orthorhombic γ-modification. The β-modification is characterized by improved mechanical properties, in particular improved impact strength and improved resistance to stress cracking.

Typically, crystallization in the β-modification is achieved by adding specific β-nucleating agents, such as quinacridone pigments, which are disclosed in EP 0 177 961 A2. A further well-known class of β-nucleating agents is Group II salts of dibasic organic acids.

US-A-5,231,126 discloses that β-nucleation can be achieved by the admixing of isotactic polypropylene with a two-component β-nucleating agent consisting of a mixture of a dibasic acid with an oxide, hydroxide or acid salt of a Group II metal. Suitable examples of dibasic acids are pimelic acid, azelaic acid, o-phtalic acid, terephthalic acid and isophthalic acid and the like. Suitable oxide, hydroxides or acid salts of Group II metals are compounds comprising magnesium, calcium, strontium or barium, with typical examples including calcium carbonate or other carbonates.

A drawback of the two-component β-nucleating agent disclosed in this prior art document, however, is the insufficient reproducibility of the effect achieved, since the melt mixing of the two-component β-nucleating agent with the polypropylene may lead to varying results, due to the influence of parameters such as melt temperature, shear conditions, compounding time, presence of impurities and water, etc. EP 0682066 A1 discloses in a comparative example a process for increasing the content of β-modification in polypropylene, comprising a step wherein polypropylene is melted in the presence of calcium stearate and pimelic acid in order to form an *in situ* generated β-Nucleating agent. While the comparative example in this prior art document reveals a high amount of β-modification, the reproducibility of the melt mixing and melt reaction process is highly unsatisfactory, as demonstrated further by comparative examples contained in the present application showing, under similar conditions, an amount of β-modification being substantially 0.

It furthermore has to be emphasized that several of the starting materials employed in the prior art for preparing the β-nucleating agents are rather expensive, so that improvements, in particular with respect to a reliable and reproducible use of those pricey materials is required.

Efforts accordingly have been made to prepare more reliable systems for achieving β-nucleation, based on dibasic-organic acids and Group II metal compounds.

EP 0 682 066 A1 discloses such an attempt for achieving a more reliable β-modification. This document describes that improvements can be achieved by employing a one-component β-nucleating agent, produced by reacting 1 mol of dicarboxylic acid with 1 mol calcium carbonate in an aqueous ethanol containing solution at 60 to 80°C. This reaction yields the calcium salt of the dicarboxylic acid which is obtained in the form of a fine precipitate which can be isolated by filtration. Thereafter, the product is dried and may be used as β-nucleating agent.

The drawback of this one-component β-nucleating agent, namely the calcium salt of the dicarboxylic acid is, on the one hand, the fact that the obtained precipitate contains 1 mol of crystal water which decreases the effect of β-nucleation. The removal of this crystal water is only achievable under severe conditions which, however, increases the cost of the additive, since additional heating steps are required. A further drawback is the fact that the one-component β-nucleating agent is obtained in the form of a fine precipitate, which gives rise to problems during filtration. In particular, fine precipitates are a major drawback when considering the scale up of the synthesis, since fine precipitates will lead to a drastic decrease of filtration efficiency.

It furthermore has to be taken into account that dibasic organic acids are rather expensive raw materials, so that it would be advantageous to reduce the amount of dibasic organic acid required for β-nucleation.

With respect to the other β-nucleating agents, such as the above-mentioned quinacridone pigments, it has to be noted further that same give rise, even when added in low amounts only, to a discoloration of the polypropylene. Even at amounts of below 10 ppm, the color of the polypropylene containing such β-nucleating agents shows a discoloration being discernible with the eye, typically a light red color. This is, of course, a further drawback which decreases the value of the nucleated polypropylene.

WO 02/078924 discloses metal salts of hexahydrophthalic acid as nucleating additives for crystalline thermoplastics, which are used in order to improve stiffness and transparency. These nucleating agents accordingly are α-nucleating agents, which is readily apparent from the disclosure in this prior art document referring to the nucleating agents also as clarifying agents.

## Object of the present invention

In view of the drawbacks identified above, it is the object of the present invention to provide a novel and improved β-nucleating agent, which enables the preparation of polypropylene in the β-modification without giving rise to the drawbacks associated with the conventional β-nucleating agents such as discoloration. Furthermore, the effects as obtained should be reproducible and the use of costly raw materials, such as dibasic organic acids should be reduced as much as possible. The β-nucleating agent furthermore should be obtainable by a process which also enables the preparation of the β-nucleating agent at a larger scale. It furthermore would be advantageous if costly and time consuming further treatments, such as removal of crystal water could be avoided.

## Brief description of the Invention

The above objects are solved with a process in accordance with claim 1. Preferred embodiments are disclosed subclaims 2 to 7 as well as in the following specification. The present invention furthermore provides a β-nucleating agent as defined in claim 8. Preferred embodiments are again presented in dependent subclaims 9 to 12. Finally, the present invention also provides a polypropylene composition, as defined in claim 13.

## Detailed description of the present Invention

As defined in claim 1, the present invention provides a process for preparing a β-nucleating agent, comprising the step of treating a mixture comprising a dibasic organic acid and an oxide, hydroxide or acid salt of a Group II metal at a temperature of above 120°C.

It is important in this respect to emphasize that the process, in accordance with the present invention as defined in claim 1 and as further illustrated herein, is a reaction in which the Group II metal compound is reacted directly with a dibasic organic acid at the given temperature, i.e. the reaction occurs without the additional presence of any further solvents or molten materials, such as a molten polymer material. The reaction of the Group II metal compound with the dibasic organic acid is a reaction of these two components absent of any further components (with the exception of air or other gaseous atmospheres which do not interfere with the reaction). In particular, the process in accordance with the present invention is not a process which is carried out in solution or in a molten matrix of polymer. The Group II metal compound and the dibasic organic acid are, in the process in accordance with the present invention, brought directly into contact with each other and are reacted at the temperatures defined herein.

The dibasic acid to be employed in accordance with the present invention may be selected among dibasic organic acids comprising four or more carbon atoms. Preferred examples of dibasic acids are pimelic acid, suberic acid, azelaic acid, o-phthalic acid, terephthalic acid as well as isophthalic acid. These acids may be used singly or in any desired admixture. Preferred are in particular pimelic acid, suberic acid as well as azelaic acid.

The oxide, hydroxide or acid salt of a Group II metal to be employed in accordance with the present invention typically is a compound comprising calcium, magnesium, strontium or barium or mixtures thereof. Preferred are in particular calcium compounds. Suitable examples include calcium hydroxide as well as calcium carbonate, as well as magnesium carbonate, strontium carbonate and barium carbonate. Preferred in particular is calcium carbonate.

The dibasic organic acid and the compound of a Group II metal are employed as mixtures comprising higher molar amounts of the Group II metal compounds than of the dibasic organic acid. A particularly preferred embodiment of the present invention, is the use of a mixture comprising a high excess of the Group II metal compound, which is the less expensive component for the preparation of the β-nucleating agent. Suitable ratios of Group II metal compound to dibasic organic acid are as follows:

**Weight ratio**: 10:5-0.1, in particular, 10:3-0.5; more preferably, 10:2-1
**Mole ratio**: 10:5-0.1, in particular, 10:3-0.5; more preferably, 10:2-1.

In the method in accordance with the present invention, it is, in particular, preferred when the two components, prior to the above-identified heat treatment are admixed intensively, preferably using a ball mill, a roll mill or a corresponding device. This premixing typically is carried out at a temperature of from 10 to 40°C, preferably 20 to 25°C.

As identified above, the components for the preparation of the β-nucleating agents are in accordance with the technical teaching of the present invention subjected to a heat treatment at a temperature of 120°C or more, preferably 140°C or more and most preferably 150°C or more. A particular suitable treatment temperature is about 160°C. The heat treatment may be carried out for a period of time suitable for giving rise to the desired one-component β-nucleating agent in accordance with the present invention and typical examples of treatment times are 30 minutes or longer, more preferably 1 hour or longer, and in particular 1.5 hours or longer, such as about 2 hours. This heat treatment is typically carried out at ambient pressure, either in the presence of air or in the presence of an inert gas, such as nitrogen. The upper limit for the temperature is not critical, however, the temperature should not be above the decomposition temperature of the organic dibasic acid.

The heat treatment may be carried out in any desired and suitable device, including stirred vessels and ball mills as well as fluidized bed reactors. In particular, the use of fluidized bed reactors is preferred in accordance with the present invention. These devices, which are well known to the skilled person, enable an intimate mixing of the two components and further allows for a continuous process, a further advantage in particular regarding the scale-up of the process in accordance with the present invention.

The heat treatment in accordance with the present invention is carried out in the absence of solvents or other liquid reaction media. The heat treatment, according to the results as illustrated in the following examples, gives rise to a solid-state reaction (i.e. a reaction wherein the Group II metal compound is present in solid state, while the organic acid may be present in molten form) between the dibasic acid and the Group II metal compound, yielding the corresponding salt of the dibasic acid and, depending upon the ration of starting compounds, residual Group II metal compound. The present invention accordingly enables the preparation of pure Group II metal salt of the dibasic acid, when employing an equimolar mixture, while at the same time, also enabling the preparation of any desired mixture of salt of the dibasic acid and, depending upon the ration of starting compounds, residual Group II metal compound. Surprising in the context of the present invention is on the one hand the fact that the heat treatment in accordance with the present invention gives rise to a solid state reaction, while on the other hand even the not equimolar mixtures yield, after heat treatment, a highly efficient $\beta$-nucleating agent, although only low contents of dibasic acid salt are present.

After the heat treatment, the obtained one-component $\beta$-nucleating agent may be subjected to further post treatments, in particular after having been cooled down to room temperature. In this respect, it is in particular preferred to subject the obtained one-component $\beta$-nucleating agent to a further milling treatment to obtain a fine powder having a weight average particle size of from 1 to 10 $\mu$m, preferably 2 to 7 $\mu$m, and in particular 3 to 5 $\mu$m.

The obtained $\beta$-nucleating agent is a one-component $\beta$-nucleating agent comprising the Group II metal compound as well as the dibasic organic acid in reacted form. Namely, the dibasic organic acid surprisingly reacts during the heat treatment with the Group II metal compound forming a one-component $\beta$-nucleating agent comprising, depending on the compositional ratio, Group II metal compound and the Group II metal salt of the dibasic acid. Free dibasic acid typically is no longer contained in the one-component $\beta$-nucleating agent after heat treatment.

In view of the fact that the process for preparing the one-component $\beta$-nucleating agent does not involve any reaction steps in aqueous solutions or other liquid reaction media, the drawback of obtaining products comprising crystal water as identified above in connection with the prior art can be avoided. Since the process of the present invention is a solid state reaction time and cost intensive filtering processes can be avoided. Furthermore, depending on the compositional ratio, it is possible to obtain one-component $\beta$-nucleating agents comprising only a minor amount of Group II metal salt of the dibasic organic acid in admixture with remaining amounts of Group II metal compound. However, as will be explained further below, even these one-component $\beta$-nucleating agents, which can be considered as small particles of Group II metal compound being modified at the surface thereof with the Group II metal salt of the dibasic organic acid, achieve a reproducible and highly efficient $\beta$-nucleation in polypropylene compositions, so that it is possible to obtain the desired $\beta$-modification of polypropylene with lower amounts of dibasic organic acid required, compared to the prior art $\beta$-nucleating agents.

The process in accordance with the present invention furthermore is reliable, simple to carry out and allows a scaling-up by using suitably adapted standard devices, such as fluidized bed reactors. Taking into account that highly efficient and reliable $\beta$-nucleating agents may be produced using the present invention it is readily apparent that the present invention adds a significant improvement to the art. Surprising in this respect is in particular the fact that a solid-state reaction is achieved, since previously the salt of the dibasic acid could only be prepared in solution using water as solvent, while attempts to use other polar solvents, such as ethanol, did not give rise to any reaction.

The $\beta$-nucleating agent obtained in accordance with the present invention may be used in particulate form, preferably as fine powder, when adding the $\beta$-nucleating agent to the polypropylene to be nucleated, but the present invention also envisages the use of the $\beta$-nucleating agent in the form of a master-batch, i.e. in the form of a compound comprising a polymeric matrix, preferably a polypropylene, and a rather high concentration of the $\beta$-nucleating agent.

The $\beta$-nucleating agent obtained by the present invention is used in polypropylene compositions in amounts of from 0.001 to 5 wt.-%, preferably 0.01 to 2 wt.-%, such as from 0.05 to 1 wt.-% (calculated on the basis of the polypropylene content). Thereby a degree of $\beta$-modification in the polypropylene of up to 80% or more as determined by DSC can be achieved, even in the presence of other additives, such as fillers, stabilizers, lubricants etc. The amount of $\beta$-modification may also be expressed as k value according to Tumer-Jones, and the present invention achieves k values of up to 0.94 or more (such as 0.97). Regarding the k value according to Turner-Jones and the calculation thereof reference is made to the corresponding description in EP 0 682 066 A1, incorporated herein by reference.

The polypropylene to which the $\beta$-nucleating agent in accordance with the present invention may be added may be a homopolymer as well as a copolymer, including random copolymers as well as block copolymers. The polypropylenes are typically stereoregular polypropylenes, such as isotactic polypropylenes as well as elastomeric polypropylenes having a degree of stereo regularity of preferably 80% or more. Stereoregularity is preferably determined by $^{13}$C-NMR spectroscopy in solution as described e.g. by Busico et al. in Macromolecules 28 (1995) 1887-1892, taking the isotactic pentad regularity (mmmm) as measure of stereoregularity.

In accordance with a further aspect of the present invention, the present invention provides a novel one-component β-nucleating agent, obtainable by a process as identified in the present application.

Preferred embodiments as described above in connection with the process of the present invention likewise also apply with respect to the one-component β-nucleating agent in accordance with the present invention.

Furthermore, the present invention provides a novel β-nucleating agent comprising the solid phase reaction product of a Group II metal compounds selected among oxides, hydroxides and acid salts, with a dibasic organic acid, wherein the β-nucleating agent comprises particles of Group II metal compound as identified above modified at the surface with a Group II metal salt of a dibasic organic acid.

These novel β-nucleating agents are obtained in accordance with the a process in accordance with the present invention, wherein an excess of Group II metal compound is employed, preferably an excess as defined further below, so that the respective particles of the Group II metal compound are only modified at the surface with the dibasic organic acid, resulting in the Group II metal salt of a dibasic organic acid, present on the surface of the particles of the Group II metal compound. These Group II metal salts of a dibasic organic acid are formed on the surface of the Group II metal compound particles where the dibasic organic acid comes into contact with said particles. The obtained β-nucleating agent still displays a highly satisfactory ability to provide the β-modification when used as nucleating agent in polypropylene while employing a rather minor amount of the expensive starting material dibasic organic acid; compared in particular with the prior art techniques where Group II metal salts of dibasic organic acids are formed in chemical reactions, for example, in solution. This embodiment of the present invention accordingly is a highly cost effective and easy to handle β-nucleating agent, the beneficial effects of which are in particular are illustrated by Example No. 3 as shown below. Even with low amounts of the β-nucleating agent according to Example No. 3 satisfactory amounts of β-nucleation can be obtained, as given and explained in the examples contained in the present application.

Preferred embodiments as identified above in connection with the process in accordance with the present invention likewise also apply with respect to the β-nucleating agent defined herein.

In particular the β-nucleating agent comprises a ratio of Group II metal compound to dibasic organic acid as follows:

| **Weight ratio**: | 10:5-0.1, in particular, 10:3-0.5; more preferably, 10:2-1 |
| **Mole ratio**: | 10:5-0.1, in particular, 10:3-0.5; more preferably, 10:2-1. |

A particularly preferred β-nucleating agent comprises calcium carbonate and the calcium salt of a dibasic acid, preferably pimelic acid, on the surface of the individual calcium carbonate particles. This structure is the result of the solid state reaction yielding the β-nucleating agent of the present invention. This structure and composition can be confirmed by IR-spectroscopy as well as by microscopic techniques enabling the evaluation of surface portions of individual particles.

Such β-nucleating agents, in particular the embodiments prepared using a large excess of Group II metal compound, do give rise to fully satisfactory and reproducible contents of the desired β-modification in polypropylene compositions while reducing drastically the required amount of dibasic acid needed for the β-nucleation. This is a vast improvement compared with the prior art, yielding either not well reproducible β-nucleation or requiring higher amounts of the costly dibasic acid.

Finally the present invention provides a polypropylene composition, comprising a polypropylene and a β-nucleating agent as described herein. The polypropylene may be any kind of polypropylene, as outlined above, including in particular isotactic polypropylenes, such as homopolymers and copolymers, typically copolymers comprising only small amounts of comonomers, such as copolymers described in the art as random copolymers. The composition as provided by the present invention comprises the β-nucleating agent as described herein in amounts yielding the desired content of β-modification, typically of from 0.001 to 5 wt.-%, preferable 0.01 to 2 wt.-%, and in embodiments 0.05 to 1 wt.-%, based on the content of polypropylene. Other usual additives and fillers may be present as well in typical amounts. The polypropylene composition in accordance with the present invention typically displays a k value according to Turner-Jones of above 0.5, and the resent invention enables the provision of polypropylene compositions showing k values of as much as 0.85 or more. This corresponds to a content of β-modification as determined by DSC of up to 80% or more.

The following examples illustrate the present invention further:

Examples

Mixtures 1-3 were prepared by intensively mixing of calcium carbonate (supplied by Fluka, Art. No. 21060) and Pimelic acid (supplied by Fluka, Art. No. 80500) at room temperature, then putting the mixtures into a convection oven at 160°C for two hours. After cooling down to room temperature all mixtures were milled to a fine powder with an average particle size of 3-5μm.

| No. | CaCO$_3$ [gram] | Pimelic acid [gram] | Comment | Heat Treatment |
|---|---|---|---|---|
| 1 | 10.00 | 10.00 | wt. by wt. | 2h/160°C |
| comparative example 2 | 10.01 | 16.02 | Equimolar mixture | 2h/160°C |
| 3 | 10.00 | 1.00 | wt. by wt. | 2h/160°C |
| * comparative example | | | | |

Another mixture has been prepared in the following way (mixture 4):

16.02 g pimelic acid was dissolved in 100ml of pure ethanol (supplied by Fluka, Art. No. 02883) and heated up to 60°C. then 10,01g CaCO$_3$ (supplied by Fluka, Art. No. 21060) were added and stirred for two hours. No reaction to Ca-Pimelate at all could be detected, since no development of CO$_2$ was seen. The suspension was filtrated, dried at 110°C and identified by IR-spectroscopy. The precipitate could be identified as pure CaCO$_3$ but no Ca-Pimelate could be identified.

Mixtures 1 to 4 were evaluated regarding β-nucleation in polypropylene:

Recipes and results:

A polypropylene homopolymer powder (MFR(230/2.16): 0.2g/10min) was mixed with 0.5% pentaerythrityl-tetrakis(3-(3',5'-di-tert. butyl-4-hydroxyphenyl)-propionate (supplied as Irganox 1010, Ciba SC), 0.10% tris (2,4-di-t-butylphenyl) phosphite (supplied as Irgafos 168, Ciba SC) and 0.10% Ca-Stearate (supplied as Ca-Stearate S, Faci) and the mixtures 1 to 4 as mentioned above were added in amounts as indicated in the following table and extruded with a Theyson TSE 24 twin screw extruder at a melt temperature of 230°C. The obtained products were evaluated and the results are shown in the following table:

| Nucleating agent | Tm beta [°C] | Tm alpha [°C] | Hm beta [J/g] | Hm alpha [J/g] | Tk [°C] | Beta-content [%] |
|---|---|---|---|---|---|---|
| no (CE1) | 146.7 | 163.7 | 2.8 | 97.3 | 112.2 | <5 |
| 0.1% mixture 1 | 150.8 | 163.8 | 70.1 | 34.9 | 123.1 | 66.8 |
| 0.2% mixture 1 | 152.1 | 165.3 | 69.3 | 28.6 | 122.8 | 70.8 |
| 0.1% mixture 2 | 151.9 | 168.1 | 79.2 | 24.1 | 121.7 | 76.7 |
| 0.2% mixture 2 | 151.8 | 168.2 | 78.0 | 21.4 | 122.6 | 78.5 |
| 0.1% mixture 3 | 152.0 | 164.9 | 69.5 | 35.7 | 124.2 | 66.1 |
| 0.2% mixture 3 | 152.3 | 165.5 | 61.8 | 37.9 | 124.8 | 62.0 |
| 0.2% mixture 4 (CE2) | 148.2 | 164.8 | 3.1 | 96.9 | 113.7 | <5 |

The β-content was calculated as the ratio between to heat of fusion for the melting peak of the β-phase and the total heat of fusion (β-phase + β-phase):

$$\beta\text{-content} = H_{\beta\text{-phase}}/(H_{\beta\text{-phase}} + H_{\beta\text{-phase}})$$

An additional calculation of the β-content was done via the Turner-Jones equation (A.Tumer-Jones et al.,

Makromol Chem., 75 (1964) 134).

| Nucleating agent | k-value |
|---|---|
| 0.2% mixture 1 | 0.82 |
| 0.2% mixture 2 | 0.71 |
| 0.2% mixture 3 | 0.59 |
| 0.2% mixture 4 | 0.04 |

The β-content of all mixtures containing one of the mixture 1-3 is significantly higher than 60% by DSC, which shows a highly efficiency as β-nucleating agent of these mixtures.

The comparative example 1 (CE1) without β-nucleating agents and the comparative example (CE2) with the solution prepared mixture 4 show no significant β-content.

The k-value according to Turner-Jones is significantly higher than 0.5 for all samples prepared using the mixtures of the present invention, which indicates a high content of the β-modification in the samples and hence a high efficiency of the inventive β-nucleating agent.

From the inventive examples it can be clearly seen that a reduction of the relative amount of the dibasic acid from an equimolar ratio (mixture 2) to the very high weight ratio of 10 (mixture 3) does not compromise the efficiency as β-nucleating agent, even at identical concentrations.

Comparative examples 3 to 5 were prepared according to DE 36 10 644 (the German language equivalent to US 5,231,126) by mixing of pimelic acid with $CaCO_3$ and Ca-stearate respectively (1:1 by weight) without any further treatment.

The same polypropylene homopolymer powder (MFR(230/2.16): 0.2g/10min) as used in the previous examples was mixed with 0.5% pentaerythrityl-tetrakis(3-(3',5'-di-tert. butyl-4-hydroxyphenyl)-propionate (supplied as Irganox 1010, Ciba SC), 0.10% tris (2,4-di-t-butylphenyl) phosphite (supplied as Irgafos 168, Ciba SC) and 0.10% Ca-Stearate (supplied as Ca-Stearate S, Faci) and the $CaCO_3$/pimelic acid mixtures prepared according to DE 36 10 644 as mentioned above and extruded with a Theyson TSE 24 twin screw extruder at a melt temperature of 230°C. The compositions and the results of subsequent evaluations of the products obtained are given in the following table:

| Nucleating agent | Tm Beta [°C] | Tm alpha [°C] | Hm beta [J/g] | Hm alpha N/g] | Tk [°C] | Beta content [%] |
|---|---|---|---|---|---|---|
| 0.1% pimelic acid/ CaSt (CE3) | 165.4 | 1.6 | 106.6 | 117.0 | <2 |
| 0.1% pimelic acid/ $CaCO_3$ (CE4) | 166.9 | 0.0 | 109.4 | 123.8 | 0.0 |
| 0.2% pimelic acid/ $CaCO_3$ (CE5) | 167.5 | 0.0 | 101.5 | 125.5 | 0.0 |

The comparative examples CE3 to CE5 do not show any indication of an increase of the content of the β-modification of polypropylene as measured by DSC.

Comparative example 6:

With BE 50 (commercial PP-homopolymer from Borealis without any nucleating agent, MFR(230/2.16): 0.2g/10min) 2mm thick compression molded plaques were prepared and the k-value according to Turner-Jones was measured. The k-value was <0.01, which indicates basically no presence of the β-modification of PP. This composition corresponds to the one of comparative example 1 given above.

**Claims**

1. Process for preparing a β-nucleating agent, comprising the step of heat treating a Group II metal compound with a dibasic organic acid at a temperature of 120°C or more, wherein the process is carried out in the absence of any solvents or molten materials, such as a molten polymer material.

2. Process in accordance with claim 1, wherein the Group II metal compound is a calcium compound.

3. Process in accordance with claim 1 or 2, wherein the dibasic organic acid is selected among pimelic acid, suberic acid, and azelaic acid.

4. Process in accordance with claim 1, 2 or 3, wherein the heat treatment is carried out at a temperature of 150°C or more.

5. Process in accordance with any one of claims 1 to 4, wherein heat treatment is carried out in a fluidized bed reactor.

6. Process in accordance with any one of claims 1 to 5, wherein the heat treatment is carried out for a duration of 1.5 hours or more.

7. Process in accordance with any one o claims 1 to 6, wherein the dibasic acid is employed in an amount giving rise to a weight ratio or mole ratio of Group II metal compound to dibasic acid of 10:5-0.1.

8. β-nucleating agent, obtainable by any of the processes according to claims 1 to 7, wherein the Group II metal compound and the dibasic organic acid are employed in a ratio comprising a molar excess of the Group II metal compound.

9. β-nucleating agent in accordance with claim 8, wherein the mole ratio or the weight ratio of Group II metal compound to dibasic organic acid is 10:1.

10. β-nucleating agent in accordance with claims 8 or 9, comprising a solid phase reaction product of the Group II metal compound and the dibasic organic acid, wherein the β-nucleating agent comprises particles of Group II metal compound being modified at the surface thereof with a Group 11 metal salt of a dibasic organic acid.

11. β-nucleating agent in accordance with claim 10, wherein the Group II metal compound is calcium carbonate and the dibasic organic acid is pimelic acid.

12. β-nucleating agent in accordance with claim 11 or 12, wherein the mole ratio or the weight ratio of Group II metal compound to Group II metal salt of the dibasic organic acid is 10:1.

13. Polypropylene composition, comprising a polypropylene and a β-nucleating agent according to any one of claims 8 to 12.

**Patentansprüche**

1. Verfahren zum Herstellen eines β-Nukleierungsmittels, umfassend den Schritt des Wärmebehandelns einer Gruppe II - Metallverbindung mit einer zweibasischen organischen Säure bei einer Temperatur von 120 °C oder mehr, wobei das Verfahren in der Abwesenheit von irgendwelchen Lösungsmitteln oder geschmolzenen Materialien, wie ein geschmolzenes Polymermaterial, durchgeführt wird.

2. Verfahren gemäß Anspruch 1, wobei die Gruppe II - Metallverbindung eine Kalziumverbindung ist.

3. Verfahren gemäß Anspruch 1 oder 2, wobei die zweibasische organische Säure ausgewählt ist unter Pimelinsäure, Suberinsäure oder Azelainsäure.

4. Verfahren gemäß Anspruch 1, 2 oder 3, wobei die Wärmebehandlung bei einer Temperatur von 150 °C oder mehr durchgeführt wird.

5. Verfahren gemäß einem der Ansprüche 1 bis 4, wobei die Wärmebehandlung in einem Fließbettreaktor durchgeführt wird.

6. Verfahren gemäß einem der Ansprüche 1 bis 5, wobei die Wärmebehandlung für eine Dauer von 1,5 Stunden oder mehr durchgeführt wird.

7. Verfahren gemäß einem der Ansprüche 1 bis 6, wobei die zweibasische Säure in einer Menge eingesetzt wird, die zu einem Gewichtsverhältnis oder Molverhältnis an Gruppe II - Metallverbindung zu zweibasischer Säure von 10: 5-0,1 führt.

8. β-Nukleierungsmittel, erhältlich durch eines der Verfahren gemäß Anspruch 1 bis 7, wobei die Gruppe II - Metallverbindung und die zweibasische organische Säure in einem Verhältnis eingesetzt werden, das einen molaren Überschuss der Gruppe II - Metallverbindung umfasst.

9. β-Nukleierungsmittel gemäß Anspruch 8, wobei das Molverhältnis oder Gewichtsverhältnis an Gruppe II - Metallverbindung zu zweibasischer Säure 10:1 ist.

10. β-Nukleierungsmittel gemäß Anspruch 8 oder 9, umfassend ein Festphasenreaktionsprodukt der Gruppe II - Metallverbindung und der zweibasischen organischen Säure, wobei das β-Nukleierungsmittel Partikel der Gruppe II - Metallverbindung umfasst, die an ihrer Oberfläche mit einem Gruppe II - Metallsalz der zweibasischen organischen Säure modifiziert sind.

11. β-Nukleierungsmittel gemäß Anspruch 10, wobei die Gruppe II - Metallverbindung Kalziumcarbonat ist und die zweibasische organische Säure Pimelinsäure ist.

12. β-Nukleierungsmittel gemäß Anspruch 11 oder 12, wobei das Molverhältnis oder Gewichtsverhältnis an Gruppe II - Metallverbindung zu Gruppe II - Metallsalz der zweibasischen organischen Säure 10:1 ist.

13. Polypropylenzusammensetzung, umfassend ein Polypropylen und ein β-Nukleierungsmittel gemäß einem der Ansprüche 8 bis 12.

**Revendications**

1. Procédé pour préparer un agent de β-nucléation, comprenant l'étape consistant à traiter à la chaleur un composé d'un métal du Groupe II avec un acide organique dibasique à une température de 120°C ou plus, lequel procédé est mis en oeuvre en l'absence de tout solvant ou matériau fondu, tel qu'un matériau polymère fondu.

2. Procédé selon la revendication 1, dans lequel le composé d'un métal du Groupe II est un composé du calcium.

3. Procédé selon la revendication 1 ou 2, dans lequel l'acide organique dibasique est choisi parmi l'acide pimélique, l'acide subérique, et l'acide azélaïque.

4. Procédé selon la revendication 1, 2 ou 3, dans lequel le traitement à la chaleur est effectué à une température de 150°C ou plus.

5. Procédé selon l'une quelconque des revendications 1 à 4, dans lequel le traitement à la chaleur est effectué dans un réacteur à lit fluidisé.

6. Procédé selon l'une quelconque des revendications 1 à 5, dans lequel le traitement à la chaleur est effectué pendant 1,5 heures ou plus.

7. Procédé selon l'une quelconque des revendications 1 à 6, dans lequel l'acide dibasique est employé en une quantité conduisant à un rapport en poids ou un rapport en moles du composé d'un métal du Groupe II sur l'acide dibasique de 10/5-0,1.

8. Agent de β-nucléation pouvant être obtenu par l'un quelconque des procédés selon les revendications 1 à 7, dans lequel le composé d'un métal du Groupe II et l'acide organique dibasique sont employés en un rapport comprenant un excès molaire du composé d'un métal du Groupe II.

9. Agent de β-nucléation selon la revendication 8, dans lequel le rapport en moles ou le rapport en poids du composé d'un métal du Groupe II sur l'acide organique dibasique est de 10/1.

10. Agent de β-nucléation selon la revendication 8 ou 9, comprenant un produit de réaction en phase solide du composé d'un métal du Groupe II et de l'acide organique dibasique, lequel agent de β-nucléation comprend des particules de composé d'un métal du Groupe II qui ont été modifiées sur leur surface avec un sel d'un métal du Groupe II d'un acide organique dibasique.

**11.** Agent de β-nucléation selon la revendication 10, dans lequel le composé d'un métal du Groupe II est le carbonate de calcium et l'acide organique dibasique est l'acide pimélique.

**12.** Agent de β-nucléation selon la revendication 11 ou 12, dans lequel le rapport en moles ou le rapport en poids du composé d'un métal du Groupe II au sel d'un métal du Groupe II de l'acide organique dibasique est de 10/1.

**13.** Composition de polypropylène comprenant un polypropylène et un agent de β-nucléation selon l'une quelconque des revendications 8 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0177961 A2
- US 5231126 A
- EP 0682066 A1
- WO 02078924 A
- DE 3610644

**Non-patent literature cited in the description**

- **BUSICO et al.** *Macromolecules,* 1995, vol. 28, 1887-1892